# FASCICULE DE BREVET EUROPEEN

(11) **EP 0 395 478 B1**
(45) Date de publication et mention de la délivrance du brevet: **19.01.1994**
(21) Numéro de dépôt: 90401082.4
(22) Date de dépôt: 20.04.1990
(51) Int. Cl.: A61B 3/11

(54) **Pupillomètre automatique entièrement statique**
Ganzstatischer automatischer Pupillometer
Automatic pupillometer entirely static

(30) Priorité: 25.04.1989 FR 8905478
(43) Date de publication de la demande: 31.10.1990
(73) Titulaire: ESSILOR INTERNATIONAL Compagnie Générale d'Optique, F-94028 Créteil Cédex (FR)
(72) Inventeur: Hennequin, Jean-Claude, F-77400 Lizy-Sur-Ourcq (FR); Massart, Christian, F-93390 Clichy-Sous-Bois (FR)
(74) Mandataire: CABINET BONNET-THIRION

(56) Documents cités:
- EP-A- 0 305 238
- DE-U- 8 812 095
- GB-A- 2 152 232

## Description

L'invention se rapporte à un pupillomètre pour la mesure d'écartement des pupilles d'un sujet, en vue notamment de l'adaptation d'une paire de lunettes, comportant un boîtier avec une face frontale généralement plane, apte à s'appliquer sur le visage d'un sujet dans une position relative repérée, en sorte qu'un axe principal normal à la face frontale et un plan d'horizon contenant cet axe principal passent respectivement sensiblement à équidistance et par les centres des yeux du sujet, ceux-ci étant à distance fixée de la face frontale et cette face frontale étant ajourée à hauteur des yeux ; dans le boîtier, à proximité de la face frontale, une lentille maîtresse convergente dont l'axe optique coïncide avec l'axe principal; un point source lumineux situé optiquement sur l'axe principal à une position telle qu'il détermine, pour le sujet qui le fixe à travers la lentille maîtresse, une distance d'observation choisie, la lumière émise par le point source formant, sur les centres des cornées du sujet une paire de reflets respectifs ponctuels, dits reflets cornéens ; et des images optiques de ces reflets cornéens venant en coïncidence avec une paire de divisions discrètes respectives d'une échelle de mesure étendue suivant le plan d'horizon normalement à l'axe principal ; des moyens pour identifier les divisions en coïncidence avec la paire d'images respectives de reflets cornéens ; et des moyens pour traduire et afficher l'écart pupillaire correspondant à une paire de divisions identifiées.

On notera que l'axe principal du pupillomètre est dirigé suivant une normale au plan général de la face du sujet, et que le plan d'horizon se réfère à l'attitude du sujet avec la tête droite, en fixant l'horizon. Par ailleurs, la face frontale correspond sensiblement à la portion normale des verres de lunettes.

Le document de brevet FR-A-1 506 352 décrit un pupillomètre qui comporte l'optique de base, à savoir la lentille maîtresse convergente, un point source situé optiquement sur l'axe principal du boîtier pour définir une distance d'observation pour le sujet, et former au centre des cornées du sujet des points lumineux. Dans ce pupillomètre, la lentille maîtresse est déplaçable suivant l'axe optique principal, à partir d'une position origine proche de la face frontale où le point source est optiquement situé au foyer de la lentille maîtresse, ce qui correspond à une distance d'observation infinie. Un oeilleton est ménagé dans le boîtier sur l'axe principal à l'opposé de la face frontale, en coïncidence optique avec le point source. Deux alidades munies d'un trait vertical sont disposées sensiblement dans le plan de la face frontale et sont déplaçables suivant le plan d'horizon. Un observateur, regardant par l'oeilleton, amène les traits verticaux des alidades respectivement à passer par le reflet cornéen du sujet. Des réglettes graduées, solidaires des alidades, permettent de lire l'écart pupillaire.

Ce pupillomètre, dont le principe optique est excellent, souffrait d'une imprécision dans la manoeuvre des alidades et la lecture des réglettes graduées. Le document de brevet FR-A-2 538 239 décrit un pupillomètre, comportant la même optique de base, dont les alidades sont remplacées par des matrices à cristaux liquides au moins adressables par colonnes. Une colonne commutée fait apparaître un trait vertical, l'adresse de la colonne étant représentative de l'abscisse du trait sur l'intersection du plan de la face frontale et du plan d'horizon. L'observateur déplace ainsi les traits verticaux en commandant en comptage ou décomptage des générateurs d'adresse. Un microprocesseur, qui pilote toute l'électronique, traduit en écart pupillaire la différence des abscisses des traits verticaux exprimées par les adresses des colonnes correspondantes, en tenant compte de la distance qui sépare les deux matrices, et des pas de ces matrices. Cet écart pupillaire est affiché sur un dispositif d'affichage numérique. On notera que ce pupillomètre permet, si les matrices de cristaux liquides sont adressables en ligne, de déterminer la portion de la partie inférieure d'une monture de lunettes par rapport au centre de la cornée, dans un plan vertical.

Ce pupillomètre, dont la précision et la commodité d'emploi sont nettement supérieures à celles du pupillomètre selon FR-A-1 506 352, exige cependant encore l'intervention d'un observateur pour régler la coïncidence des traits verticaux repères de l'échelle de mesure avec les images de reflets cornéens, d'autant qu'il faut régler indépendamment les coïncidences pour l'oeil droit et l'oeil gauche. Outre les erreurs dues à l'équation personnelle de l'observateur, les distractions du regard du sujet, qui cesse de fixer l'image du point source pendant le réglage de coïncidence, risquent de faire perdre de la précision à la mesure.

Il apparaissait donc souhaitable de disposer d'un pupillomètre capable de déterminer les coïncidences d'images de reflets cornéens avec des divisions d'une échelle de mesure, et d afficher l'écart pupillaire, sans l'intervention d'un observateur dans la mesure proprement dite.

Le document de brevet FR-A-2 618 666 décrit un pupillomètre automatique dans lequel, pour chaque oeil, il est formé un réseau de reflets cornéens avec des lignes lumineuses parallèles étendues perpendiculairement au plan d'horizon, et en association un référentiel de position. Une combinaison de lentilles et de point de source définit une distance d'observation pour le sujet. En coïncidence optique avec le point source est disposé un miroir tournant, dont l'axe de rotation est perpendiculaire au plan d'horizon. Les images des reflets cornéens, et des référentiels de position, repris par le miroir tournant et convenablement focalisés défilent devant une fente, parallèle à l'axe du miroir, pratiquée dans un masque derrière lequel est disposé un détecteur photo-électrique. L'analyse du signal fourni par le détecteur photo-électrique permet de situer les reflets cornéens par rapport aux référentiels de position. On notera que, pratiquement, pour chaque oeil, seul le reflet cornéen correspondant à la ligne du réseau la plus proche du centre de la cornée forme une image reprise par le miroir tournant, en raison du caractère divergent du miroir formé par la cornée.

Ce pupillomètre ne nécessite pas le réglage de réticules ou d'alidades par un observateur, et est donc automatique ou impersonnel, cette expression signifiant que n'intervient pas l'équation personnelle d'un observateur. Cependant il nécessite l'utilisation d'un miroir tournant, à vitesse de rotation régulière : en outre l'exploitation des signaux issus du détecteur photo-électrique est relativement complexe car, dans une première phase, le référentiel de mesure, détecté, est mis en mémoire, tandis que, dans une seconde phase, les reflets cornéens sont situés par rapport au référentiel mémorisé.

Le document de brevet FR-A-2 620 927 décrit un appareillage pour mesurer les facteurs nécessaires à l'adaptation de verres optiques sur une monture. Cet appareillage comporte essentiellement une caméra optique à élément photosensible à transfert de charge, un télémètre pour enregistrer la distance du sujet à la caméra, et un ordinateur avec un logiciel d'exploitation. Une vue de la face du sujet, munie de la monture de lunettes est prise, et la distance de prise de vue est enregistrée pour définir l'échelle de l'image. Les signaux d'image sont traités par l'ordinateur pour en extraire les paramètres intéressants. En fait, le véritable processus de mesure est mis en oeuvre par le logiciel, dont aucune description n'est avancée.

L'invention a pour objet un pupillomètre, qui reprend très largement l'optique de base selon FR-A-1 506 352 et FR-A-2 538 239, mais qui est automatique et donc impersonnel, et en outre entièrement statique.

A cet effet l'invention propose un pupillomètre pour la mesure d'écartement des centres des pupilles d'un sujet, en vue notamment de l'adaptation d'une paire de lunettes, comportant un boîtier avec une face frontale généralement plane, apte à s'appliquer sur le visage d'un sujet dans une position relative repérée, en sorte qu'un axe principal normal à la face frontale et un plan d'horizon contenant cet axe passent respectivement sensiblement à équidistance et par les centres des yeux du sujet, ceux-ci étant à distance fixée de la face frontale et celle-ci étant ajourée à hauteur des yeux ; dans le boîtier, à proximité de la face frontale une lentille maîtresse convergente dont l'axe optique coïncide avec l'axe principal ; un point source lumineux situé optiquement sur l'axe principal à une position telle qu'il détermine, pour le sujet qui le fixe à travers la lentille maîtresse, une distance d'observation choisie, la lumière émise par le point source formant, sur le centre des cornées du sujet des reflets respectifs ponctuels, dits reflets cornéens ; et des images optiques de ces reflets cornéens venant en coïncidence avec une paire de divisions discrètes respectives d'une échelle de mesure étendue suivant le plan d'horizon normalement à l'axe principal ; des moyens pour identifier des divisions en coïncidence avec la paire d'images respectives des reflets cornéens, et des moyens pour traduire et afficher l'écart pupillaire correspondant à une paire de divisions identifiées, pupillomètre caractérisé en ce qu'il comporte une optique d'objectif apte à former, dans un plan image où s'étend l'échelle de mesure, une paire d'images réelles des reflets cornéens vus à travers la lentille maîtresse, l'échelle de mesure comprenant au moins un dispositif photo-électique avec un réseau d'éléments sensibles constituant divisions de l'échelle, des moyens d'analyse séquentielle du réseau identifiant le rang, dans le réseau, des éléments sur lesquels se forme l'image d'un reflet cornéen.

Ici l'échelle de mesure est physiquement stable, et l'optique d'objectif détermine une relation stable entre l'écart pupillaire et l'écart des images réelles sur l'échelle de mesure. L'analyse séquentielle du réseau d'éléments photo-électriques sensibles ne fait intervenir aucun déplacement de pièces mobiles.

Etant donné que l'écart pupillaire en vision de près peut se déduire de l'écart pupillaire en vision à l'infini avec une bonne précision, la distance entre le centre de rotation de l'oeil et la cornée ne variant que peu d'un sujet à l'autre et n'introduisant que des variations du second ordre de l'écart pupillaire en vision de près, le point source sera, d'une façon générale, situé optiquement au foyer de la lentille maîtresse.

En disposition préférée le dispositif photo-électrique est un dispositif semi-conducteur dit à transfert de charge où un réseau de grilles de champ convenablement polarisées forme un réseau de puits de potentiel où s'accumulent des porteurs de charge formés par l'impact de photons sur une région voisine d'une cible photosensible. Pour l'analyse, des trains d'impulsion appliquée aux grilles de champ font glisser les parois des points de potentiel dans une direction de balayage, par pas du réseau. Les charges accumulées entre deux balayages dans le puits de potentiel sont ainsi amenées séquentiellement dans une région d'analyse, où elles créent un signal, correspondant en amplitude, à la charge accumulée dans chacun des puits de potentiel, et en rang dans l'analyse, au rang de position du puits de potentiel considéré.

De préférence, dans un but de simplicité, le dispositif à transfert de charge est un réseau à une dimension. En effet, dans la mesure où seul l'écart pupillaire est recherché, il ne servirait à rien de mettre en oeuvre un dispositif à transfert de charge en matrice à deux dimensions, qui requiert une analyse en double balayage, correspondant à une analyse de signal de télévision.

Selon un premier mode d'exécution, l'optique d'objectif comprend une paire de lentilles convergentes disposées symétriquement par rapport à l'axe principal, chacune avec un axe optique dans le plan d'horizon parallèlement à l'axe principal, et un dispositif photo-électrique avec un réseau de cellules est associé à chaque lentille d'objectif.

L'écart entre les axes optiques des lentilles de la paire d'optique d'objectif sera normalement réglé sur la moyenne des écarts pupillaires rencontrés. Les images des reflets cornéens focalisées sur les réseaux photo-électriques vont s'écarter des axes optiques des lentilles d'objectif d'un angle, vu du centre optique de la lentille, égal à l'angle, vu du même centre optique, que forme le rayon issu de l'image virtuelle, formée par la lentille maîtresse du reflet cornéen correspondant avec l'axe optique de la lentille d'objectif. Dans cette disposition, donc, on détermine essentiellement la différence entre l'écart pupillaire réel du sujet, et l'écart pupillaire moyen choisi.

Selon un autre mode d'exécution, l'optique d'objectif comprend une lentille d'entrée, convergente, dont l'axe optique coïncide avec l'axe principal du pupillomètre, à une distance de la lentille maîtresse telle que leur combinaison convergente présente un plan focal, parallèle au plan de la face frontale, et à la distance fixée des yeux à cette face, et une lentille de sortie convergente avec un axe optique en coïncidence avec l'axe principal, un dispositif photo-électrique avec un réseau d'éléments photosensibles étant disposé dans un plan focal de la lentille de sortie du côté de celle-ci opposé à la lentille d'entrée d'objectif.

Dans cette disposition, on utilise un seul dispositif photo-électrique sur lequel on projette une image de la face du sujet, où seuls les reflets cornéens apparaissent. On effectue donc, à une échelle déterminée, une mesure réelle de l'écart pupillaire, et non une détermination de la différence entre l'écart pupillaire réel et l'écart pupillaire moyen. Si dans ce second mode d'exécution, l'erreur aléatoire relative provenant d'une imprécision sur le rang des éléments photosensibles intéressés, est supérieure à celle que donne le premier mode d'exécution, il n'y a pas d'erreur systématique provenant d'une imprécision sur la distance qui sépare les axes optiques de la paire de lentilles d'objectif.

Dans le second mode d'exécution, les trois lentilles sont centrées sur l'axe principal. Le point source doit donc être physiquement à l'écart de cet axe principal. Aussi dispose-t-on, de préférence, une lame semi-réfléchissante entre la lentille maîtresse et la lentille d'entrée d'objectif : cette lame semi-réfléchissante forme un angle avec le plan d'horizon qu'elle coupe suivant une normale à l'axe principal, en sorte de définir un rayon principal réfléchi, le point source étant optiquement situé sur ce rayon principal réfléchi à un point conjugué du foyer de la lentille maîtresse.

De préférence alors un miroir plan est disposé parallèlement à la lame semi-réfléchissante sur le trajet du rayon principal réfléchi, le point source lumineux étant disposé physiquement sur un rayon, parallèle à l'axe principal, déterminé par la réflexion sur le miroir plan du rayon principal réfléchi.

On préférera que la lame semi-réfléchissante forme avec le plan d'horizon un angle dièdre de 45°.

Des caractéristiques secondaires et des avantages de l'invention ressortiront d'ailleurs de la description qui va suivre, à titre d'exemple, en référence aux dessins annexés dans lesquels :
La figure 1 est une vue schématique en coupe d'un pupillomètre selon l'invention ;
La figure 2 est un schéma rappelant le mode de formation d'un reflet cornéen ;
La figure 3 est une coupe, suivant un plan d'horizon, d'un premier mode d'exécution de l'invention ;
La figure 4 est une coupe, suivant un plan d'horizon d'un deuxième mode d'exécution de l'invention ;
La figure 5 est une coupe, suivant un plan vertical, du pupillomètre selon la figure 4 ;
La figure 6 est un schéma des circuits électroniques qui font suite aux agencements optiques dans les pupillomètres selon l'invention.

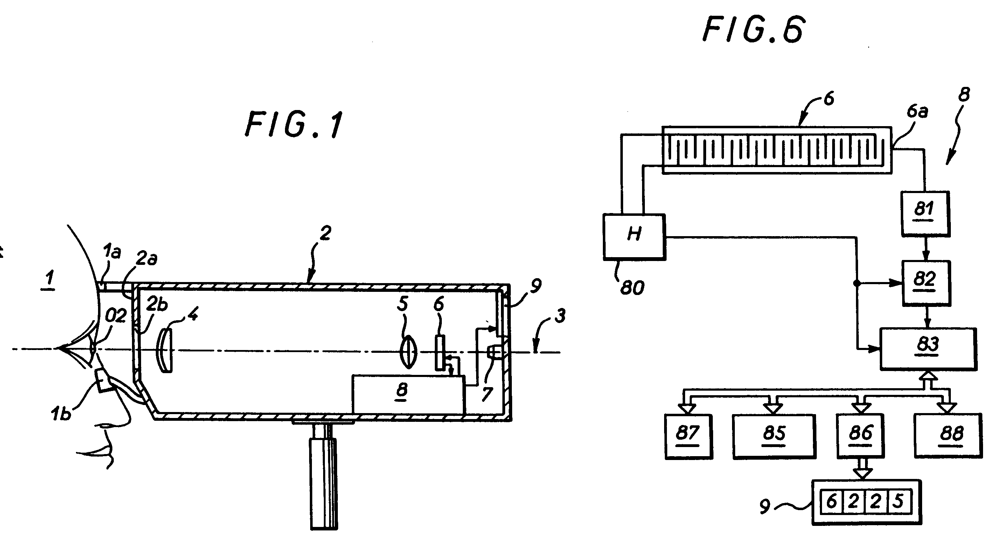

Selon la forme de réalisation choisie et représentée figure 1, le pupillomètre comporte un boîtier 2, avec une face frontale 2a généralement plane et perpendiculaire à un axe principal 3. De cette face frontale 2a partent un appui frontal 1a, et un jeu de plaquettes nasales 1b tels que, en appui sur la face d'un sujet 1, la face frontale 2a soit globalement parallèle à la face du sujet 1, que l'axe principal 3 passe à équidistance des yeux du sujet 1, et qu'un plan d'horizon passant par l'axe principal et perpendiculaire au plan de la figure passe par le centre des yeux (02 pour l'oeil droit du sujet, seul apparent sur la figure).

La face frontale 2a comprend des ajours 2b, en face des yeux du sujet, qui correspondent sensiblement à l'emplacement de verres de lunettes dans la monture.

Dans le boîtier 2, à proximité de la face frontale 2a est placée une lentille convergente 4, qui sera dite lentille maîtresse, dont l'axe optique coïncide avec l'axe principal 3 du pupillomètre, et dont le foyer, du côté opposé à la face frontale 2b coïncide avec un point source lumineux 7 situé contre une face parallèle et opposée à la face frontale 2a. On comprend que, vu à travers la lentille maîtresse 4 par le sujet 1, le point source lumineux apparaît à l'infini, les rayons visuels partant des yeux du sujet étant parallèles à l'axe principal 3.

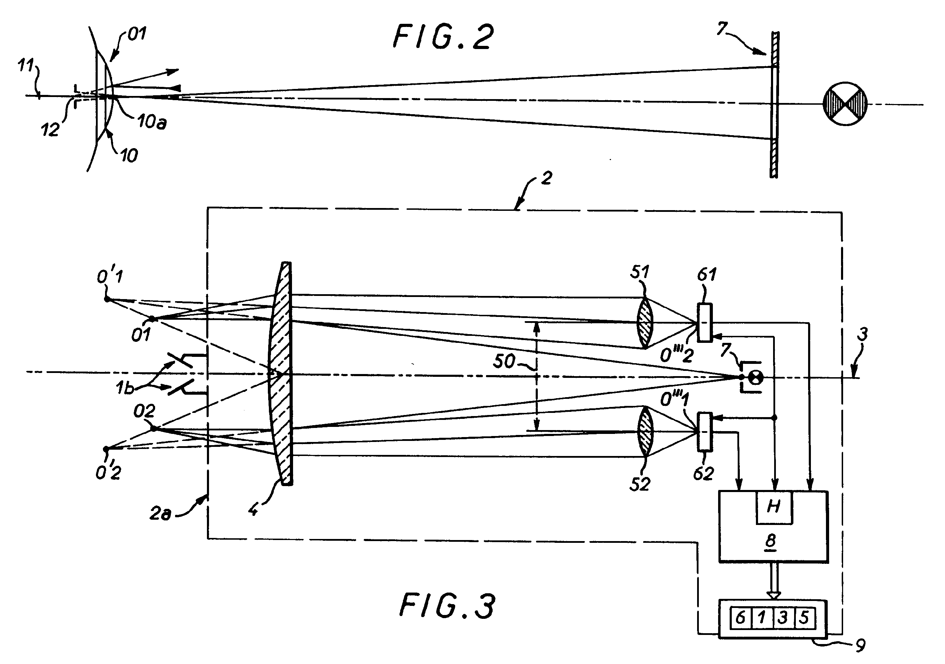

La lumière émise par le point source 7 se reflète au centre de la cornée de chacun des yeux du sujet 1, sous forme d'un point lumineux, comme on le précisera à propos de la figure 2.

Les images virtuelles des reflets cornéens déterminées par la lentille 4, constituent pour un dispositif objectif 5 des objets réels à quoi correspondent des images réelles dans un plan, perpendiculaire à l'axe principal, où se trouve placée une échelle de mesure 6, étendue dans le plan d'horizon. Cette échelle de mesure 6 comporte des dispositifs à transfert de charge, dont le contenu du réseau d'éléments photosensibles est analysé en séquence par un dispositif de pilotage et lecture 8, avec des moyens de calculs capables d'afficher l'écart pupillaire. Cela sera décrit de façon détaillée en référence aux figures 3 à 6.

En se reportant à la figure 2, l'oeil O1 comporte, en légère saillie du globe oculaire, une cornée 10 en forme sensiblement de calotte sphérique avec son centre en 11. La cornée 10, bien que transparente, est partiellement réfléchissante et constitue pour des rayons lumineux extérieurs, un miroir sphérique convexe 10 dont le foyer 12 est à mi-distance entre le centre 11 et le sommet 10a de la calotte sphérique. Pour ce miroir sphérique 10, le point source 7, ou plus exactement son image à travers la lentille 4, est situé à l'infini. L'image de ce point source 7 donnée par le miroir sphérique convexe 10 est située dans son plan focal 12, avec une ouverture angulaire égale à l'ouverture angulaire apparente du point source 7. Pour fixer les idées, si le point source présente un diamètre d'ouverture de 2 mm et la lentille maîtresse 4 une distance focale de 300 mm, en prenant pour rayon de courbure de la cornée 9 mm , soit une focale de miroir de 4,5 mm, le diamètre de l'image virtuelle dans le plan 12 sera de 0,03 mm.

Selon le mode d'exécution représenté figure 3, où le boîtier 2 est représenté schématiquement, les yeux du sujet gauche et droit prennent les positions O1 et O2 respectivement, lorsque la face du sujet se place contre la face frontale 2a, avec les plaques nasales 1b en appui sur le nez du sujet. La lentille maîtresse 4 est disposée dans le boîtier 2, à proximité de la face frontale 2a, avec son axe optique en coïncidence avec l'axe principal 3 du pupillomètre. Le point source 7, représenté sous forme d'une source lumineuse derrière une ouverture, est situé sur l'axe principal 3, au foyer de la lentille 4 du côté de celle-ci opposé à la face frontale 2a. Le sujet, fixant le point source 7 à travers la lentille 4, le voit rejeté à l'infini, et les axes optiques des yeux O1 et O2 sont parallèles à l'axe optique principal 3, et dans le plan d'horizon qui est ici le plan de figure. Les images du point source 7 données par les cornées du sujet sont des images virtuelles en O1 et O2, comme on l'a expliqué en référence à la figure 2.

Pour un observateur (fictif) placé du même côté de la lentille 4 que le point source 7, cette lentille 4 donne des reflets cornéens O1 et O2 des images virtuelles O'1 et O'2, respectivement. Pour construire graphiquement les emplacements des images virtuelles O'1 et O'2, on se souviendra que des rayons parallèles à l'axe issu de O1 et O2 sont déviés pour passer par le foyer, et que des rayons passant par le centre optique de la lentille 4 ne sont pas déviés. Ainsi O'1 et O'2 sont alignés avec O1 et O2 sur des droites passant par le centre optique de la lentille 4, et sont alignés avec la projection de 01 et 02 sur le plan principal de la lentille 4 sur des droites passant par le foyer 7.

Une optique d'objectif, constituée de deux lentilles convergentes 51 et 52, disposées symétriquement par rapport à l'axe principal 3 avec leurs axes optiques dans le plan d'horizon (ou plan de figure) et à une distance 50 l'une de l'autre, donne des images réelles 0'1 et O'2, constituant objets réels, des images réelles O'''1 et O'''2 respectivement, sur une paire de barrettes à transfert de charge 61 et 62 respectivement.

Ces barrettes sont étendues dans le plan d'horizon perpendiculairement à l'axe optique principal.

Les images réelles O'''1 et O'''2 sont alignées avec les images O'1 et O'2 respectivement sur des droites passant par le centre optique des lentilles 51 et 52 respectivement. On a représenté les faisceaux, issus des reflets cornéens en O1 et O2, qui viennent converger sur les barrettes à transfert de charge 61 et 62, par leur rayon central et les rayons marginaux.

On a choisi la distance 50 pour correspondre à la valeur moyenne d'écartement pupillaire des sujets. On notera que l'optique d'objectif fait inverseur, de sorte que les déplacements O'''1 et O'''2 par rapport à l'axe optique des lentilles 61 et 62 respectivement sont en direction inverse des écarts de O'1 et O'2 par rapport à ces axes optiques. On remarquera que la distance 50 n'est pas la valeur moyenne des écarts pupillaires, mais la valeur moyenne de l'écart des images O'1 et O'2, qui tient compte du grandissement introduit par la lentille maîtresse 4.

Comme il a déjà été rappelé, dans les cellules sur lesquelles se forment les images réelles O'''1 et O'''2 s'accumulent des charges libérées dans le semi-conducteur sous l'impact des photons. L'analyse séquentielle du contenu des puits de potentiel des éléments des barrettes, en réponse à des impulsions d'horloge forme des signaux où chaque élément est caractérisé par son rang de la barrette. Compte tenu du pas de réseau de la barrette, l'analyse séquentielle identifie, avec le rang de l'élément où s'est formée l'image O'''1 ou O'''2, l'écart en dimension de l'image par rapport à une origine centrée.

Il est donc possible de calculer, dans une unité de pilotage et de calcul 8, l'écart pupillaire à partir des paramètres optiques de grandissement, déterminables, de l'écart 50 entre les lentilles d'objectif 51 et 52, et du rang des cellules frappées par les images O'''1 et O'''2, l'écart pupillaire qui s'inscrira sur l'afficheur 9. On peut également calculer et afficher l'écart entre le centre de chaque pupille et l'axe principal 3, au cas où les yeux seraient dissymétriques. Dans un cas pratique, la lentille 4 présente une distance focale de 250 mm, et les lentilles 51 et 52 des distances focales de 25 mm. Les yeux O1 et O2 sont placés à 70 mm du plan principal de la lentille, tandis que les centres optiques des lentilles 51 et 52 sont à 200 mm de ce plan principal. La distance qui sépare les lentilles 51, 52 des barrettes à transfert de charge 61 et 62 est de 27 mm. Les calculs de grandissement se font par les formules classiques bien connues en optique.

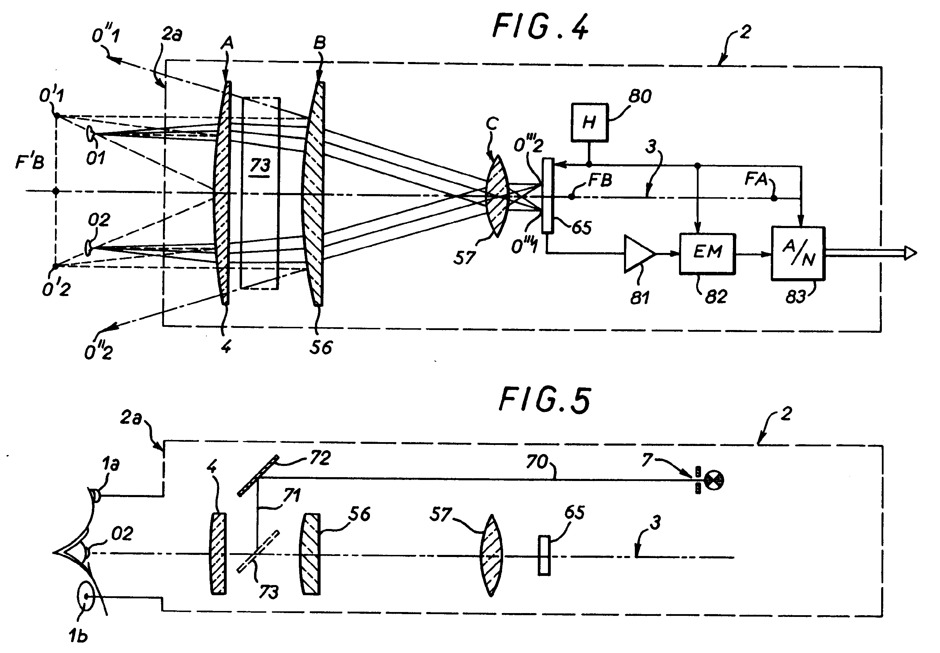

Le mode d'exécution représenté figures 4 et 5 va comporter une seule barrette à transfert de charge 65, et toute l'optique va être centrée sur l'axe principal.

Dans le boîtier 2 est disposée une lentille maîtresse 4, ou A, convergente dont le foyer FA est situé sur l'axe principal 3, cette lentille maîtresse étant à proximité de la face frontale. Le point source 7 (voir figure 5), est situé optiquement au foyer de la lentille maîtresse 4, mais est décalé géométriquement perpendiculairement au plan d'horizon (plan de la figure 4) par une lame semi-réfléchissante 73 qui coupe à 45° le plan d'horizon suivant une perpendiculaire à l'axe principal 3, et un miroir 72 parallèle à la lame 73, de sorte que, vu dans un plan vertical (plan de la figure 5), le rayon central du trajet optique entre le point source 7 et la lentille maîtresse 4 suit d'abord une parallèle à l'axe principal 3 jusqu'au miroir 72, puis une perpendiculaire 71 à cet axe 3 entre le miroir 72 et la lame semi-réfléchissante 73, pour se mettre en coïncidence avec l'axe principal 3 au-delà. Pour le sujet, dont les yeux gauche et droit sont respectivement en O1 et O2, le point source 7 apparaît à l'infini sur l'axe principal 3 dans le plan d'horizon. Pour un observateur placé du côté de la lentille 4 opposé à celui de la face frontale 2a, cette lentille 4 donne des reflets cornéens en O1 et O2, des images virtuelles en O'1 et O'2, respectivement de la façon qui a été expliquée à propos de la figure 3.

Une optique d'objectif comporte une lentille d'entrée 56 ou B convergente, d'étendue comparable à celle de la lentille maîtresse 4, dont l'axe optique coïncide avec l'axe principal 3 ; son foyer est en FB. La lame semi-réfléchissante se trouve située entre la lentille maîtresse 4 et la lentille d'entrée d'objectif 56. La focale de la lentille 56 et sa distance à la lentille maîtresse 4 sont telles que la lentille composée formée des lentilles 4 et 56 ait un plan focal dans lequel sont placés les yeux O1 et O2 du sujet. On rappelle que, optiquement, les yeux sont les reflets cornéens. On peut exprimer cette condition en remarquant, de façon équivalente, que les images virtuelles O'1 et O'2 des reflets cornéens O1, O2 données par la lentille 4 sont dans le plan focal de la lentille d'entrée 56, plan focal qui coupe l'axe optique principal en F'B.

La lentille 56 forme des images virtuelles O'1, O'2 formant objets réels, des images O''1, O''2 rejetées à l'infini. Les directions de ces images à l'infini O''1, O''2, peuvent être déterminées graphiquement en notant que les rayons parallèles à l'axe principal 3 issus de O'1 et O'2 sont réfractés par la lentille d'entrée 56 pour passer par FB.

L'optique d'objectif comprend encore une lentille de sortie 57 (ou C), dont l'axe optique coïncide avec l'axe principal 3. Cette lentille de sortie 57 forme, à partir des images à l'infini O''1, O''2, des images réelles O'''1 et O'''2 dans son plan focal, où se trouve placée une barrette à transfert de charge 65 étendue dans le plan d'horizon (plan de la figure 4) perpendiculairement à l'axe optique principal.

Sur la figure 4 on a représenté les faisceaux, définis par le rayon central et les rayons marginaux, qui partent des reflets cornéens O1, O2 pour aboutir sur la barrette à transfert de charge 65 en O'''1 et O'''2, respectivement.

Le rapport entre la distance O'''1-O'''2 et l'écart pupillaire O1, O2 se détermine à partir des distances focales des lentilles 4, 56 et 57, et de leurs positions sur l'axe optique principal 3. Dans un exemple la distance entre les yeux O1, O2 et la lentille maîtresse 4 est de 70 mm, la longueur focale de cette lentille 4 de 300 mm, la distance entre centres optiques des lentilles maîtresse 4 et d'entrée d'objectif 56 de 50 mm, avec une distance focale de la lentille d'entrée 56 de 140 mm, la distance entre centres optiques des lentilles d'entrée 56 et de sortie 57 d'objectif de 98 mm, et la distance focale de lentille de sortie d'objectif 57 de 25 mm. Avec ces valeurs, on trouve un rapport de grandissement d'environ 0,3. On remarquera que la distance entre centres optiques des lentilles d'entrée 56 et de sortie 57 de l'optique d'objectif ne joue qu'un rôle du second ordre, puisque la lentille de sortie 57 donne, dans son plan focal des images d'objets à l'infini, et l'écartement de ces images dépend de la distance focale et de l'écart angulaire des objets O''1, O''2. Aussi la distance entre centres optiques des lentilles d'objectif intervient pour répartir l'obliquité des faisceaux par rapport aux dioptres rencontrées successivement, et réduire ainsi les aberrations.

L'analyse séquentielle de la barrette à transfert de charges 65 résulte du transfert des contenus des puits de potentiel de la barrette au rythme des impulsions d'horloge 80. Les signaux d'analyse sont amplifiés et filtrés en 81, mis en forme par un circuit d'échantillonnage et maintien 82, et mémorisés par un convertisseur analogique numérique 83 qui leur associe une adresse de rang correspondant au rang de l'élément photosensible du réseau de barrette 65, pour être traités par un microprocesseur.

En se reportant à la figure 6, on voit que l'horloge 80 dirige sur la barrette à transfert de charge deux trains de signaux à une paire de réseaux interdigités de grille, qui avec un troisième réseau convenablement polarisé, forment entre eux des parois de puits s'élargissant, par glissement de leur paroi située du côté de la région d'analyse 6a, puis se rétrécissent par glissement de leur autre paroi, en se décalant ainsi d'un pas à chaque fois.

Comme on l'a décrit précédemment, l'arrivée séquentielle, au cours de la phase d'analyse, des charges accumulées dans les différents puits de potentiel au cours d'une phase précédente d'accumulation, dans la région d'analyse fournit un signal représentatif temporellement des quantités de lumière reçues par chaque cellule du réseau en séquence spatiale. Ce signal est amplifié et filtré par un étage 81, puis mis en forme par un étage d'échantillonnage et maintien 82, qui est sensible à la tension d'entrée pendant la durée d'une impulsion courte au rythme d'horloge, et qui maintient jusqu'à l'apparition de l'impulsion suivante la tension détectée. Le convertisseur 83 transforme le signal analogique délivré par l'étage 82 en signal numérique, auquel est associée une adresse de rang.

Ce signal numérique complexe est traité par un microprocesseur 85 auquel sont associées une mémoire 87, et une sortie d'affichage 86, qui est reliée à un afficheur numérique. Un sélecteur 88 permet de choisir les modes de calculs, les contrôles et étalonnages nécessaires. Toute l'organisation autour du microprocesseur 85 vise des commodités d'exploitation du pupillomètre, qui sortent du cadre de la présente invention.

Mais il va de soi, que dans une mémoire permanente, sont enregistrées les données, telles que grandissement optique, pas des barrettes à transfert de charge, et écartement des axes des lentilles d'objectif dans le cas du pupillomètre selon la figure 3. La présente description est suffisamment détaillée pour qu'un homme de métier soit capable de mener à bien toute la partie relative à l'exploitation des déterminations de coïncidence des images de reflets cornéens avec les divisions des échelles de mesure.

Par ailleurs, il va de soi que la présente invention n'est pas limitée aux exemples décrits, mais en embrasse toutes les variantes de réalisation, dans le cadre des revendications.

Notamment, la lentille maîtresse 4 pourrait être mobile parallèlement à l'axe principal 3, pour mesurer l'écart pupillaire pour une distance d'observation rapprochée, et non à l'infini. Les échelles de mesure pourraient être réalisées avec des dispositifs photo-électriques autres que des dispositifs à transfert de charge, même si ces dispositifs à transfert de charge ont une sensibilité particulièrement intéressante en raison du mécanisme d'accumulation de charges dans les puits de potentiel.

En outre les dispositifs photo-électriques utilisés pour la réalisation des échelles de mesure, singulièrement les dispositifs à transfert de charge, pourraient avoir une structure de matrice à lignes et colonnes. On pourrait alors prévoir, outre la mesure de l'écart pupillaire, la mesure de la distance verticale entre le bas de la monture de verre de lunettes et le reflet cornéen correspondant.

## Revendications

1. Pupillomètre pour la mesure d'écartement des centres (01 , 02) des pupilles d'un sujet, en vue notamment de l'adaptation d'une paire de lunettes, comportant un boîtier (2) avec une face frontale (2a) généralement plane, apte à s'appliquer sur le visage d'un sujet (1) dans une position relative repérée, en sorte qu'un axe principal (3) normal à la face frontale (2a) et un plan d'horizon contenant cet axe passent respectivement sensiblement à équidistance et par les centres (O1, O2) des yeux du sujet, ceux-ci étant à distance fixée de la face frontale (2a) et celle-ci étant ajourée (2b) à hauteur des yeux ; dans le boîtier, à proximité de la face frontale (2a) une lentille maîtresse (4) convergente dont l'axe optique coïncide avec l'axe principal (3) ; un point source (7) lumineux situé optiquement sur l'axe principal (3) à une position telle qu'il détermine, pour le sujet (1) qui le fixe à travers la lentille maîtresse (4), une distance d'observation choisie, la lumière émise par le point source (7) formant, sur le centre (O1, O2) des cornées du sujet (1) des reflets respectifs ponctuels, dits reflets cornéens ; et des images optiques (O'''1, O'''2) de ces reflets cornéens venant en coïncidence avec une paire de divisions discrètes respectives d'une échelle de mesure (6) étendue suivant le plan d'horizon normalement à l'axe principal (3) ; des moyens (8) pour identifier les divisions en coïncidence avec la paire (O'''1, O'''2) d'images respectives des reflets cornéens, et des moyens (8, 9) pour traduire et afficher l'écart pupillaire correspondant à une paire de divisions identifiées, pupillomètre caractérisé en ce qu'il comporte une optique d'objectif (5; 51, 52; 56, 57) apte à former, dans un plan image où s'étend l'échelle de mesure (6; 61, 62; 65) une paire d'images réelles (O'''1, O'''2) des reflets cornéens (O1, O2) vus à travers la lentille maîtresse (4), l'échelle de mesure comprenant au moins un dispositif photo-électrique avec un réseau d'éléments sensibles constituant divisions de l'échelle (6), et des moyens (80) d'analyse séquentielle du réseau (6; 61, 62; 65) identifiant le rang, dans le réseau, des éléments sur lesquels se forme l'image (O'''1, O'''2) d'un reflet cornéen.

2. Pupillomètre selon la revendication 1, caractérisé en ce que ledit point source lumineux (7) est situé, optiquement, au foyer de la lentille maîtresse (4) en sorte que ladite distance d'observation soit infinie.

3. Pupillomètre selon l'une des revendications 1 et 2, caractérisé en ce que le dispositif photo-électrique (6) est un dispositif semi-conducteur dit à transfert de charge, avec une pluralité de puits de potentiel disposés en réseau, aptes à accumuler des charges issues d'une zone contiguë d'une cible photosensible, et des grilles de transfert aptes, en réponse à des trains d'impulsions d'analyse, à décaler les charges de puits en puits jusqu'à une région d'analyse (6a).

4. Pupillomètre selon la revendication 3, caractérisé en ce que le dispositif à transfert de charge est un réseau à une dimension ou barrette.

5. Pupillomètre selon l'une quelconque des revendications 1 à 4, caractérisé en ce que l'optique d'objectif comprend une paire de lentilles (51, 52) convergentes disposées symétriquement par rapport à l'axe principal (3), chacune avec un axe optique dans le plan d'horizon parallèlement à cet axe principal (3), un dispositif photo-électrique (61, 62) avec un réseau d'éléments photosensibles étant associé à chaque lentille (51, 52) d'objectif.

6. Pupillomètre selon l'une quelconque des revendications 1 à 4, caractérisé en ce que l'optique d'objectif comprend une lentille d'entrée (56), convergente, avec son axe optique coïncidant avec l'axe principal (3), à une distance de la lentille maîtresse (4) telle que leur combinaison convergente présente un plan focal parallèle à la face frontale (2a), à ladite distance fixée de cette face pour les yeux (O1, O2), et une lentille de sortie (57) convergente avec un axe optique en coïncidence avec l'axe principal (3), le dispositif photo-électrique (65) avec un réseau d'éléments photosensibles étant disposé dans un plan focal de la lentille de sortie (57) du côté de celle-ci opposé à celui de la lentille d'entrée (56) d'objectif.

7. Pupillomètre selon la revendication 6, caractérisé en ce qu'une lame semi-réfléchissante (73) est disposée entre la lentille maîtresse (4) et la lentille d'entrée (56) d'objectif, formant un angle dièdre avec le plan d'horizon qu'elle coupe suivant une normale à l'axe principal (3) en sorte de définir un rayon principal réfléchi (71) correspondant à un rayon venant de la lentille maîtresse (4) suivant l'axe principal (3), le point source lumineux (7) étant optiquement situé sur ce rayon principal réfléchi (71) à un point conjugué du foyer de la lentille maîtresse (4).

8. Pupillomètre selon la revendication 7, caractérisé en ce qu'un miroir plan (72) est disposé parallèlement à la lame semi-réfléchissante (73) sur le trajet du rayon principal réfléchi (71), le point source lumineux (7) étant disposé physiquement sur ce rayon (70), parallèle à l'axe principal (72) du rayon principal (71) réfléchi.

9. Pupillomètre selon l'une des revendications 7 et 8, caractérisé en ce que la lame plane semi-réfléchissante (73) forme un angle dièdre de 45° avec le plan d'horizon.

## Patentansprüche

1. Pupillometer zur Messung des Abstands der Mittelpunkte (01, 02) der Pupillen einer Person, insbesondere zur Anpassung einer Brille, mit einem Gehäuse (2) mit einer im allgemeinen ebenen Vorderfläche (2a), geeignet zur Anwendung auf das Gesicht einer Person (1) in einer relativ festgelegten Position, dergestalt, daß eine Hauptachse (3) senkrecht zu der Vorderfläche (2a) und eine diese Achse enthaltende Horizontalebene jeweils praktisch im gleichen Abstand und durch die Mittelpunkte (01, 02) der Augen der Person laufen, wobei diese in festgelegtem Abstand von der Vorderfläche (2a) sind und diese in Höhe der Augen durchbrochen (2b) ist, einer konvergierenden Hauptlinse (4) in dem Gehäuse in der Nähe der Vorderfläche (2a), deren optische Achse mit der Hauptachse (3) zusammenfällt, einem Lichtquellenpunkt (7), der optisch auf der Hauptachse (3) in einer Position derart gelegen ist, daß er für die Person (1), die ihn über die Hauptlinse (4) festlegt, einen gewählten Beobachtungsabstand bestimmt, wobei das von dem Quellenpunkt (7) ausgesandte Licht auf dem Mittelpunkt (01, 02) der Hornhaut der Person jeweils punktuelle Reflexe bildet, die Hornhautreflexe genannt werden, wobei optische Bilder (0'''1, 0'''2) der Hornhautreflexe in Koinzidenz kommen mit einem Paar von jeweiligen diskreten Unterteilungen einer Meßskala (6), die gemäß der Horizontalebene senkrecht zur Hauptachse (3) erstreckt ist, Mittel (8) zur Ermittlung der Unterteilungen in Koinzidenz mit dem Paar (0'''1, 0'''2) von jeweiligen Bildern der Hornhautreflexe, und Mitteln (8, 9) zum Übertragen und Ausgeben des Pupillenabstands entsprechend einem Paar von ermittelten Unterteilungen, dadurch gekennzeichnet, daß es eine Objektivoptik (5; 51, 52; 56, 57) aufweist, die geeignet ist, in einer Bildebene, in der sich die Meßskala (6; 61, 62; 65) erstreckt, ein Paar von reellen Bildern (0'''1, 0'''2) von Hornhautreflexen (01, 02) zu bilden, gesehen durch die Hauptlinse (4), wobei die Meßskala wenigstens eine photoelektrische Vorrichtung aufweist mit einem Netz bzw. Gitter von empfindlichen Elementen, die die Unterteilungen der Skala (6) darstellen, und Mittel (80) zur sequentiellen Analyse des Netzes (6; 61, 62; 65), welche die Stufe der Elemente in dem Gitter angeben, auf denen sich das Bild (0'''1, 0'''2) eines Hornhautreflexes bildet.

2. Pupillometer nach Anspruch 1, dadurch gekennzeichnet, daß der Lichtquellenpunkt (7) optisch im Brennpunkt der Hauptlinse (4) dergestalt gelegen ist, daß der Beobachtungsabstand unendlich ist.

3. Pupillometer nach einem der Ansprüche 1 und 2, dadurch gekennzeichnet, daß die Photoelektrische Vorrichtung (6) eine Halbleiter-Vorrichtung ist, genannt zur Ladungsübertragung, mit einer Vielzahl von Potentiallöchern, die im Netz angeordnet sind, geeignet zum Sammeln von Ladungen, die von einer photoempfindlichen Scheibe benachbarten Zone ausgegeben werden, und Übertragungsgitter, die geeignet sind, in Reaktion auf eine Analysen-Impulsreihe die Ladungen von Loch zu Loch bis zu einem Analysenbereich (6a) verschieben.

4. Pupillometer nach Anspruch 3, dadurch gekennzeichnet, daß die Vorrichtung zur Ladungsübertragung ein Netz in einer Dimension oder ein Stab ist.

5. Pupillometer nach einem der Ansprüche 1 bis 4, dadurch gekennzeichnet, daß die Objektivoptik ein Paar von konvergenten Linsen (51, 52) aufweist, die symmetrisch bezüglich der Hauptachse (3) angeordnet sind, und zwar jede mit einer optische Achse in einer Horizontalebene parallel zu der Hauptachse (3), wobei eine photoelektrische Vorrichtung (61, 62) mit einem Netz aus photoempfindlichen Elementen mit jeder Linse (51, 52) des Objektivs verbunden ist.

6. Pupillometer nach einem der Ansprüche 1 bis 4, dadurch gekennzeichnet, daß die Objektivoptik eine konvergierende Eingangslinse (56) aufweist mit einer optischen Achse, die mit der Hauptachse (3) zusammenfällt, in einem Abstand von der Hauptlinse (4) derart, daß ihre konvergente Kombination eine zur Vorderfläche (2a) parallele Brennebene aufweist, und zwar in dem festgelegten Abstand von der Fläche für die Augen (01, 02), und eine konvergierende Ausgangslinse (57) mit einer optischen Achse in Koinzidenz mit der Hauptachse (3), wobei die photoelektrische Vorrichtung (65) mit einem Netz von photoempfindlichen Elementen in einer Brennebene der Ausgangslinse (57) auf deren Seite gegenüber der Eingangslinse (56) des Objektivs angeordnet ist.

7. Pupillometer nach Anspruch 6, dadurch gekennzeichnet, daß ein halbreflektierendes Blatt (73) zwischen der Hauptlinse (4) und der Eingangslinse (56) des Objektivs angeordnet ist, und einen Flächenwinkel mit der Horizontalebene bildet, die es gemäß einer Senkrechten zu der Hauptachse (3) derart schneidet, daß ein reflektierter Hauptstrahl (71) entsprechend einem von der Hauptlinse (4) gemäß der Hauptachse (3) kommenden Strahl definiert wird, wobei der Lichtquellenpunkt (7) optisch auf dem reflektierten Hauptstrahl (71) an einem Punkt gelegen ist, der dem Brennpunkt der Hauptlinse (4) zugeordnet bzw. mit diesem verbunden ist.

8. Pupillometer nach Anspruch 7, dadurch gekennzeichnet, daß eine Spiegelebene (72) parallel zum halbreflektierenden Blatt (73) auf der Bahn des reflektierten Hauptstrahls (71) angeordnet ist, wobei der Lichtquellenpunkt (7) physisch auf dem Strahl (70) angeordnet ist, und zwar parallel zur Hauptachse (72) des reflektierten Hauptstrahls (71).

9. Pupillometer nach einem der Ansprüche 7 und 8, dadurch gekennzeichnet, daß das ebene halbreflektierende Blatt (73) einen Flächenwinkel von 45° mit der Horizontalebene bildet.

## Claims

1. A pupilometer for measuring the interpupillary distance (01, 02) of a person, particularly for fitting a pair of spectacles, comprises a box (2) with a front panel (2a) which is usually flat and which is capable of being applied to the face of a person (1) in a known relative position so that a main axis (3) normal to the front panel (2a) and a horizon plane containing this axis pass respectively substantially equidistantly between and through the centres (01, 02) of the eyes of the person which are at a fixed distance from the front panel (2a) which is aligned (2b) with the eyes; in the box, close to the front panel (2a), a convergent master lens (4) whose optical axis is coincident with the main axis (3); a point light source (7) disposed optically on the main axis (3) at a position such that it determines a selected observation distance for the person (1) who views it through the master lens (4), the light emitted by the point source (7) forming respective spot reflections, called corneal reflections, on the centre (01, 02) of the corneas of the person (1); and optical images (0'''1, 0'''2) of these corneal reflections coinciding with a pair of respective discrete divisions of a measuring scale (6) extending along the horizon plane normal to the main axis (3); means (8) for identifying the divisions which are coincident with the pair (0'''1, 0'''2) of respective images of the corneal reflections, and means (8, 9) for translating and displaying the interpupillary distance corresponding to a pair of identified divisions, the pupilometer being characterised in that it comprises an objective lens (5; 51, 52; 56, 57) capable of forming in an image plane where the measuring scale (6; 61, 62; 65) extends a pair of real images (0'''1, 0'''2) of the corneal reflections (01, 02) viewed through the master lens (4), the measuring scale comprising at least one photo-electric device with an array of sensitive elements constituting divisions in the scale (6), and means (80) for sequentially scanning the array (6; 61, 62; 65) identifying the rank in the array of the elements on which the image (0'''1, 0'''2) of a corneal reflection is formed.

2. A pupilometer according to Claim 1, characterised in that said point light source (7) is disposed optically at the focus of the master lens (4) in such a way that said observation distance is infinite.

3. A pupilometer according to one of Claims 1 and 2, characterised in that the photo-electric device (6) is a semi-conductor charge transfer device with a plurality of potential wells disposed in an array capable of accumulating charges from an area contiguous with a photosensitive target, and transfer grids which respond to series of scanning pulses and are capable of transferring the charges from well to well as far as a scanning region (6a).

4. A pupilometer according to Claim 3, characterised in that the charge transfer device is a unidimensional array or bar-like member.

5. A pupilometer according to any one of Claims 1 to 4, characterised in that the objective lens comprises a pair of convergent lenses (51, 52) disposed symmetrically relative to the main axis (3), each one with an optical axis in the horizon plane parallel to the main axis (3), a photo-electric device (61, 62) with an array of photosensitive elements being associated with each objective lens (51, 52).

6. A pupilometer according to any one of Claims 1 to 4, characterised in that the objective lens comprises a convergent entry lens (56) with its optical axis coinciding with the main axis (3), at a distance from the master lens (4) such that the convergent combination of them has a focal plane which is parallel to the front panel (2a) at said fixed spacing from the eyes (01, 02) to the panel, and a convergent exit lens (57) with an optical axis which is coincident with the main axis (3), the device comprising a photo-electric device (65) with an array of photosensitive elements being disposed in a focal plane of the exit lens (57) on the side thereof opposite the objective entry lens (56).

7. A pupilometer according to Claim 6, characterised in that a semi-reflecting mirror (73) is disposed between the master lens (4) and the objective entry lens (56), forming a dihedral angle with the horizon plane which it intersects on a normal to said main axis (3) in such a way as to define a reflected main ray (71) which corresponds to a ray coming from the master lens (4) along the main axis (3), the point light source (7) being optically disposed on this main reflected ray (71) at a point conjugate with the focus of the master lens (4).

8. A pupilometer according to Claim 7, characterised in that a plane mirror (72) is disposed parallel to the semi-reflecting mirror (73) on the path of the main reflected ray (71), the point light source (7) being disposed physically on the ray (70), parallel to the main axis (72) of the main reflected ray (71).

9. A pupilometer according to one of Claims 7 and 8, characterised in that the plane semi-reflecting mirror (73) forms a dihedral angle of 45° with the horizon plane.
